(19) Europäisches Patentamt
European Patent Office
Office européen des brevets

(11) **EP 4 495 098 A1**

(12) **EUROPEAN PATENT APPLICATION**
published in accordance with Art. 153(4) EPC

(43) Date of publication:
**22.01.2025 Bulletin 2025/04**

(21) Application number: **23770871.4**

(22) Date of filing: **16.03.2023**

(51) International Patent Classification (IPC):
$C07C\ 63/26^{(2006.01)}$    $C07C\ 63/331^{(2006.01)}$
$C07C\ 229/76^{(2006.01)}$    $C07F\ 1/08^{(2006.01)}$
$C07F\ 3/02^{(2006.01)}$    $C07F\ 5/00^{(2006.01)}$
$C07F\ 5/06^{(2006.01)}$    $C07F\ 7/28^{(2006.01)}$

(52) Cooperative Patent Classification (CPC):
**C07C 63/26; C07C 63/331; C07C 229/76;
C07F 1/08; C07F 3/02; C07F 5/00; C07F 5/06;
C07F 7/28**

(86) International application number:
**PCT/JP2023/010264**

(87) International publication number:
**WO 2023/176918 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.03.2022 JP 2022042377**

(71) Applicants:
• **Kyoto University
Kyoto-shi, Kyoto 606-8501 (JP)**
• **Sumitomo Chemical Company, Limited
Tokyo 103-6020 (JP)**

(72) Inventors:
• **KITAGAWA, Susumu
Kyoto-shi, Kyoto 606-8501 (JP)**
• **HIGUCHI, Masakazu
Kyoto-shi, Kyoto 606-8501 (JP)**
• **OTAKE, Kenichi
Kyoto-shi, Kyoto 606-8501 (JP)**
• **KIKUCHI, Yuta
Ichihara-shi, Chiba 299-0195 (JP)**

(74) Representative: **Vossius & Partner
Patentanwälte Rechtsanwälte mbB
Siebertstrasse 3
81675 München (DE)**

(54) **METAL ORGANIC FRAMEWORK**

(57)    An object of the present invention is to provide a MOF having excellent adsorptive performance for an adsorption-target material. The present invention is a metal organic framework, wherein the metal organic framework has a structure $S_{M-x}$, in the structure $S_{M-x}$, two or more metal ions are bonded to one oxygen atom in a structure $S_x$ constituted by one or more of $O^{2-}$, $OH_2$, $OH^-$, $OCH_3^-$, and $OC_2H_5^-$, and the metal organic framework has a decomposition start temperature of 200°C or higher.

EP 4 495 098 A1

**Description**

TECHNICAL FIELD

The present invention relates to a metal organic framework.

BACKGROUND ART

Metal organic frameworks are also referred to as porous coordination polymers and are a class of materials which form porous structures by coordinate bonds between metal ions and organic ligands, and are expected to adsorb and desorb gas and expected to be applied to catalysts, and the like.

For example, Patent Literature 1 discloses a metal organic framework including metal ions, first ligands, second ligands, and optional third ligands. In the metal organic framework, the metal ion is an aluminum ion; the first and second ligands are each an ion of an organic compound that includes a hetero ring with two carboxyl groups; a heteroatom and the angle formed between the carboxyl groups satisfy a predetermined condition; the third ligand is an ion of an organic compound with two carboxyl groups; and the existing proportion of each of the first to third ligands is within a predetermined range.

CITATION LIST

PATENT LITERATURE

[Patent Literature 1] Japanese Laid-Open Patent Publication No.2020-176101

SUMMARY OF THE INVENTION

PROBLEMS TO BE SOLVED BY THE INVENTION

The metal organic framework (hereinafter, may be referred to as MOF) can preferably adsorb a large amount of a target material, but previously proposed MOFs still need improvement in adsorptive performance.

Therefore, an object of the present invention is to provide a MOF having an excellent adsorptive performance for an adsorption-target material (particularly, water).

SOLUTION TO THE PROBLEMS

The present invention that can achieve the problem is as follows.

[1] A metal organic framework, wherein

the metal organic framework has a structure $S_{M-x}$,
in the structure $S_{M-x}$, two or more metal ions are bonded to one oxygen atom in a structure $S_x$ constituted by one or more of $O^{2-}$, $OH_2$, $OH^-$, $OCH_3^-$, and $OC_2H_5^-$, and
the metal organic framework has a decomposition start temperature of 200°C or higher.

[2] The metal organic framework according to [1], wherein
a ratio of O derived from the $OH_2$ to O derived from an organic ligand of the metal organic framework is 0.1 or more.
[3] The metal organic framework according to [1] or [2], wherein

the metal organic framework has a cluster structure,
the cluster structure is constituted by the structure $S_x$ and the metal ions,
in the cluster structure, the metal ions are bonded to each other via the oxygen atom in the structure $S_x$, and
the number of the metal ions in one cluster structure is 3 or 4.

[4] The metal organic framework according to any one of [1] to [3], wherein
an organic ligand of the metal organic framework includes at least one selected from the group consisting of carboxylates represented by $R(COO^-)_n$ (R represents an n-valent group, and n represents an integer of 2 or more) and an oxalate ion $(COO^-)_2$.
[5] The metal organic framework according to any one of [1] to [4], wherein

the metal ion is an ion of at least one metal selected from elements in groups 2 to 14 in the third to sixth periods in a periodic table.

ADVANTAGEOUS EFFECTS OF THE INVENTION

According to the present invention, it is possible to provide the MOF that exhibits an excellent adsorptive performance.

DESCRIPTION OF EMBODIMENTS

The present invention is directed to a MOF that has a structure $S_{M-x}$ in which two or more metal ions are bonded to one oxygen atom in a structure $S_x$ constituted by one or more of $O^{2-}$, $OH_2$, $OH^-$, $OCH_3^-$, and $OC_2H_5^-$, and has a decomposition start temperature of 200°C or higher. The structure $S_x$ can be coordinated to two or more metal ions at an oxygen atom moiety, and can act as a ligand. In addition, to $O^{2-}$, $OH_2$, $OH^-$, $OCH_3^-$, and $OC_2H_5^-$, only metal ions are bonded at an oxygen atom, and another group is not bonded. It is considered that the structure $S_{M-x}$ of the MOF can protect a structure formed by the bond between the metal ion and an organic ligand, which does not necessarily have sufficient water resistance, so that favorable water absorptive performance can be achieved. In addition, a high decomposition start temperature, that is, high thermal strength, may also be a factor to achieve excellent water absorptive performance.

Bonding of two or more metal ions to one oxygen atom in the structure $S_x$ can be determined by the minimum repeating unit of the repeating structure of the MOF, for example, the minimum repeating unit in repeating of the bond between the structure $S_x$ and the metal.

The structure $S_x$ is constituted by preferably one or more of $O^{2-}$, $OH_2$, $OH^-$, and $OCH_3^-$, and more preferably one or more of $O^{2-}$, $OH_2$, and $OH^-$. The MOF of the present invention preferably has a plurality of structures $S_{M-x}$, and the structures $S_x$ in the plurality of structures $S_{M-x}$ may be the same or different from each other.

The number of the metal ions bonded to one oxygen atom in the structure $S_x$ is two or more and is usually four or less, and the kinds of the metal ions bonded to one oxygen atom in the structure $S_x$ may be the same or different from each other.

The MOF of the present invention preferably has a cluster structure, and the cluster structure means a structure which is constituted by the metal ions and the structure $S_x$, and in which the metal ions are bonded to each other via the oxygen atom in the structure $S_x$. The MOF is a framework including the metal ions and the organic ligands (however, not including either $OCH_3^-$ or $OC_2H_5^-$, which is not bonded to any group other than metal ions), and when the MOF of the present invention has the cluster structures, the cluster structures are bonded via the organic ligands.

When the MOF has the cluster structures, the total number of $O^{2-}$, $OH_2$, and $OH^-$, which are the structures $S_x$, in one cluster structure is preferably 2 or more and 15 or less, more preferably 2 or more and 8 or less, and further preferably 4 or more and 8 or less. When the total number of $O^{2-}$, $OH_2$, and $OH^-$, which are structures $S_x$, is in the above range, the cluster structure tends to be formed and the basic skeleton of the MOF is stabilized. As a result, the water absorptive performance of the MOF is easily improved.

When the MOF includes the cluster structures, the number of the metal ions in one cluster structure is preferably 2 or more, and more preferably 3 or more, and the number of the metal ions in one cluster structure is preferably 10 or less, and more preferably 8 or less. The number of the metal ions in one cluster structure is particularly preferably 3 or 4, and in this case, the water absorptive performance is particularly improved.

The ratio of O derived from $OH_2$ in the structure $S_x$ to O derived from the organic ligand of the MOF is preferably 0.1 or more. In this case, the water absorptive performance can be improved. The ratio of O derived from $OH_2$ to O derived from the organic ligand is preferably 0.12 or more, and more preferably 0.14 or more, and the ratio may be 0.20 or less.

The ratio of O derived from $O^{2-}$ in the structure $S_x$ to O derived from the organic ligand of the MOF may be 0.2 or more and 0.4 or less. In addition, the ratio of O derived from $OH^-$ in the structure $S_x$ to O derived from the organic ligand of the MOF is preferably 0.1 or more, and more preferably 0.15 or more, and the ratio is preferably 0.25 or less.

It is particularly preferable that the MOF of the present invention has the cluster structures, the number of the metal ions in one cluster structure is 3 or 4, and the ratio of O derived from $OH_2$ to O derived from the organic ligand is 0.14 or more.

The decomposition start temperature of the MOF of the present invention is 200°C or higher, more preferably higher than 200°C, further preferably 250°C or higher, and still preferably 270°C or higher, and the decomposition start temperature is preferably 500°C or lower, more preferably 400°C or lower, and further preferably 350°C or lower.

A metal ion constituting the MOF is preferably an ion of at least one metal selected from elements in groups 2 to 14 in the third to sixth periods in the periodic table. In the present specification, the term "metal" is used to include metalloids such as Si and Ge. The metal ion constituting the MOF is preferably an ion of at least one metal selected from the group consisting of Mg, Al, Ga, In, Ti, V, Cr, Mn, Fe, Co, Ni, Cu, Zr and Hf, is more preferably an ion of at least one metal selected from the group consisting of Al, Ti, Cu, In and Mg, and is particularly preferably an ion of at least one metal selected from the group consisting of Cu, In and Mg.

An organic ligand constituting the MOF preferably includes at least one selected from the group consisting of carboxylates (carboxylato) represented by $R(COO^-)_n$ (R represents an n-valent group, and n represents an integer of 2 or more) and an oxalate ion $(COO^-)_2$. R preferably represents an aliphatic chain hydrocarbon group, an aliphatic cyclic hydrocarbon group, an aliphatic heterocyclic hydrocarbon group (group in which one or more carbon atoms of an aliphatic cyclic hydrocarbon group are substituted with heteroatoms), an aromatic hydrocarbon group, or an aromatic heterocyclic hydrocarbon group (group in which one or more carbon atoms of an aromatic hydrocarbon group are substituted with heteroatoms). The aliphatic chain hydrocarbon group may be a linear chain structure or a branched chain structure, and may be a saturated hydrocarbon group or an unsaturated hydrocarbon group. A heteroatom in the aliphatic heterocyclic hydrocarbon group or the aromatic heterocyclic hydrocarbon group is preferably nitrogen.

n is preferably 2 or more and 4 or less, more preferably 2 or more and 3 or less, and most preferably 2.

The above-described aliphatic chain hydrocarbon group, aliphatic cyclic hydrocarbon group, aliphatic heterocyclic hydrocarbon group, aromatic hydrocarbon group, and aromatic heterocyclic hydrocarbon group further include one or more functional groups X selected from the group consisting of a carboxylic acid anhydride group, -OH, $-OR^1$, $-NH_2$, $-NHR^1$, $-N(R^1)_2$, - CN, a halogeno group, -C(=S)SH, -C(=O)SH and a tautomer thereof, and $-SO_3H$. Each $R^1$ represents an alkyl group having 1 or 2 carbon atoms. As the above functional group X, -OH or $-NH_2$ is preferable, and $-NH_2$ is more preferable.

Examples of the aromatic heterocyclic hydrocarbon group include pyrazole, imidazole, thiazole, oxazole, pyridine, pyrimidine, pyridazine, pyrazine, and triazine, and pyrazole is particularly preferable.

R preferably represents at least one of an unsaturated linear hydrocarbon group, an aromatic hydrocarbon group, and a hydrocarbon group containing an aromatic ring that includes a nitrogen atom, which may have the above-described functional group X, and more preferably represents an aromatic hydrocarbon group which may have the above-described functional group X (particularly, $-NH_2$).

The number of carbon atoms in R is preferably 1 or more, and more preferably 2 or more, and is preferably 30 or less. The number of carbon atoms further preferably 6 or more, 10 or more, 15 or more, and 20 or more, in this order. The upper limit of the number of carbon atoms in R may be 28 or less, 24 or less, 18 or less, 12 or less, or 10 or less.

In particular, the organic ligand constituting the MOF preferably includes at least one selected from the group consisting of carboxylates represented by $R(COO^-)_2$ and an oxalate ion $(COO^-)_2$, and, specifically, examples thereof include a ligand in which two protons are removed from two carboxyl groups (-COOH) of a dicarboxylic acid, and a ligand in which three protons are removed from three carboxyl groups of a tricarboxylic acid.

Examples of the dicarboxylic acid include oxalic acid, succinic acid, fumaric acid, tartaric acid, 1,4-butanedicarboxylic acid, 1,4-butenedicarboxylic acid, 4-oxopyran-2,6-dicarboxylic acid, 1,6-hexanedicarboxylic acid, decanedicarboxylic acid, 1,8-heptadecanedicarboxylic acid, 1,9- heptadecanedicarboxylic acid, heptadecanedicarboxylic acid, acetylenedicarboxylic acid, 1,2-benzene dicarboxylic acid (phthalic acid), 1,3-benzene dicarboxylic acid (isophthalic acid), 2,3-pyridine dicarboxylic acid, 1,3-butadiene-1,4-dicarboxylic acid, 1,4-benzene dicarboxylic acid (terephthalic acid), 2-aminoterephthalic acid, 2,5-dihydroxyterephthalic acid, imidazole-2,4-dicarboxylic acid, 3,5-pyrazole dicarboxylic acid, 2-methylquinoline-3,4-dicarboxylic acid, quinoline-2,4-dicarboxylic acid, quinoxaline-2,3-dicarboxylic acid, 6-chloroquinoxaline-2,3-dicarboxylic acid, 4,4'-diaminodiphenylmethane-3,3'-dicarboxylic acid, quinoline-3,4-dicarboxylic acid, 7-chloro-4-hydroxyquinoline-2,8-dicarboxylic acid, diimide dicarboxylic acid, pyridine-2,6-dicarboxylic acid, 2-methylimidazole-4,5-dicarboxylic acid, thiophene-3,4-dicarboxylic acid, 2-isopropylimidazole-4,5-dicarboxylic acid, tetrahydropyran-4,4-dicarboxylic acid, perylene-3,9-dicarboxylic acid, perylene dicarboxylic acid, Pluriol E 200-dicarboxylic acid, 3,6-dioxaoctane dicarboxylic acid, 3,5-cyclohexadiene-1,2-dicarboxylic acid, octane dicarboxylic acid, pentane-3,3-carboxylic acid, 4,4'-diamino-1,1'-biphenyl-3,3'-dicarboxylic acid, 4,4'-diaminobiphenyl-3,3'-dicarboxylic acid, benzidine-3,3'-dicarboxylic acid, 1,4-bis(phenylamino)benzene-2,5-dicarboxylic acid, 1,1'-binaphthyl dicarboxylic acid, 7-chloro-8-methylquinoline-2,3-dicarboxylic acid, 1-anilino anthraquinone-2,4'-dicarboxylic acid, polytetrahydrofuran 250-dicarboxylic acid, 1,4-bis(carboxymethyl) piperazine-2,3-dicarboxylic acid, 7-chloroquinoline-3,8-dicarboxylic acid, 1-(4-carboxy)phenyl-3-(4-chloro) phenylpyrazoline-4,5-dicarboxylic acid, 1,4,5,6,7,7-hexachloro-5-norbornene-2,3-dicarboxylic acid, phenylindan dicarboxylic acid, 1,3-dibenzyl-2-oxoimidazolidine-4,5-dicarboxylic acid, 1,4-cyclohexanedicarboxylic acid, naphthalene-1,8-dicarboxylic acid, 2-benzoylbenzene-1,3-dicarboxylic acid, 1,3-dibenzyl-2-oxoimidazolidine-4,5-cis-dicarboxylic acid, 2,2'-biquinoline-4,4'-dicarboxylic acid, pyridine-3,4-dicarboxylic acid, 3,6,9-trioxaundecanedicarboxylic acid, hydroxybenzophenone dicarboxylic acid, Pluriol E 300-dicarboxylic acid, Pluriol E 400-dicarboxylic acid, Pluriol E 600-dicarboxylic acid, pyrazole-3,4-dicarboxylic acid, 2,3-pyrazine dicarboxylic acid, 5,6-dimethyl-2,3-pyrazine dicarboxylic acid, bis(4-aminophenyl)ether diimide-dicarboxylic acid, 4,4'-diaminodiphenylmethane diimide-dicarboxylic acid, bis(4-aminophenyl)sulfone diimide-dicarboxylic acid, 1,4-naphthalene dicarboxylic acid, 2,6-naphthalene dicarboxylic acid, 1,3-adamantane dicarboxylic acid, 1,8-naphthalene dicarboxylic acid, 2,3-naphthalene dicarboxylic acid, 8-methoxy-2,3-naphthalene dicarboxylic acid, 8-nitro-2,3-naphthalene dicarboxylic acid, 8-sulfo-2,3-naphthalene dicarboxylic acid, anthracene-2,3-dicarboxylic acid, 2',3'-diphenyl-p-terphenyl-4,4"-dicarboxylic acid, (diphenyl ether)-4,4'-dicarboxylic acid, imidazole-4,5-dicarboxylic acid, 4(1H)-oxothiochromene-2,8-dicarboxylic acid, 5-tert-butyl-1,3-benzene dicarboxylic acid, 7,8-quinoline dicarboxylic acid, 4,5-imidazole dicarboxylic acid, 4-cyclohexene-1,2-dicarboxylic acid, hexatriacontane dicarboxylic acid, tetradecanedicarboxylic acid, 1,7-heptanedicarboxylic acid, 5-hydroxy-1,3-benzene dicarboxylic acid, 2,5-dihydroxy-1,4-benzene dicarboxylic acid, pyrazine-2,3-dicarboxylic acid, furan-2,5-dicarboxylic acid, 1-nonene-6,9-dicarboxylic acid, eicosene dicarboxylic acid, 4,4'-dihydroxy-diphenylmethane-3,3'-dicarboxylic acid, 1-amino-4-methyl-9,10-dioxo-9,10-dihydroanthracene-2,3-dicarboxylic acid, 2,5-pyridine dicarboxylic acid, cyclohexene-2,3-dicarboxylic acid, 2,9-dichlorofluorubine-4,1 1-dicarboxylic acid, 7-chloro-3-methylquinoline-6,8-dicarboxylic acid, 2,4-dichlorobenzophenone-2',5'-dicarboxylic acid, 1,3-benzene dicarboxylic acid, 2,6-pyridine dicarboxylic acid, 1-methylpyrrole-3,4-dicarboxylic acid, 1-benzyl-1H-pyrrole-3,4-dicarboxylic acid, anthraquinone-1,5-dicarboxylic acid, 3,5-pyrazole dicarboxylic acid, 2-nitrobenzene-1,4-dicarboxylic acid, heptane-1,7-dicarboxylic acid, cyclobutane-1,1-dicarboxylic acid, 1,14-tetradecanedicarboxylic acid, 5,6-dehydronorbomane-2,3-dicarboxylic acid, 5-ethyl-2,3-pyridine dicarboxylic acid, and camphordicarboxylic acid. The dicarboxylic acid is preferably at least one selected from the group consisting of terephthalic acid and 2-aminoterephthalic acid.

Examples of the tricarboxylic acid include tricarballylic acid, aconitic acid, trimellitic acid, trimesic acid, biphenyl-3,4',5-tricarboxylic acid, and 1,3,5-tris(4-carboxyphenyl)benzene, and 1,3,5-tris(4-carboxyphenyl)benzene is preferable.

As the organic ligand constituting the MOF of the present invention, a different type of an organic ligand other than carboxylates represented by $R(COO^-)_n$ and an oxalate ion $(COO^-)_2$ may be further included, and examples of the organic ligand include at least one selected from the group consisting of urea, pyrazine, oxazole, isoxazole, thiazole, imidazole, pyrazole, 1,2,3-thiadiazole, pyridazine, pyrimidine, purine, and pteridine.

The molar ratio of the metal ion to the organic ligand (total amount thereof, in a case of a plurality of kinds of organic ligands) (metal ion/organic ligand) is preferably 0.1 or more, more preferably 0.5 or more, further preferably 1.0 or more, and the molar ratio is preferably 5 or less, more preferably 4 or less, and further preferably 3 or less.

The BET specific surface area of the MOF is preferably 200 $cm^2/g$ or more, more preferably 600 $cm^2/g$ or more, and further preferably 1000 $cm^2/g$ or more. The upper limit of the BET specific surface area is not particularly limited, and the BET specific surface area is, for example, 2000 $cm^2/g$ or less.

Next, a method for producing the MOF of the present invention will be described. The MOF of the present invention can be obtained by reacting, in a solvent, a metal compound containing the metal ion constituting the MOF and one or more kinds of organic compounds for the organic ligands constituting the MOF.

The metal compound that contains the metal ion constituting the MOF is preferably metal sulfate, metal acetate, metal nitrate, metal halide (particularly metal chloride), or metal alkoxide, is more preferably metal nitrate, metal chloride or metal alkoxide, and is further preferably metal nitrate.

The organic compound for the organic ligands constituting the MOF is preferably one or more selected from the group consisting of polyvalent carboxylic acids represented by $R(COOH)_n$ and oxalic acid. R and n are equivalent to R and n described above for the carboxylates represented by $R(COO^-)_n$, and all the above descriptions about R and n, also including preferable aspects thereof, can be referred to.

The organic compound preferably includes at least one selected from the group consisting of tricarboxylic acid and dicarboxylic acid. For specific examples thereof, the tricarboxylic acid and the dicarboxylic acids described for the carboxylates represented by $R(COO^-)_n$ and preferable ranges thereof can be referred to.

As the organic compound for the organic ligands, in addition to one or more selected from the group consisting of the polyvalent carboxylic acids represented by $R(COOH)_n$ and oxalic acid, at least one selected from the group consisting of urea, pyrazine, oxazole, isoxazole, thiazole, imidazole, pyrazole, 1,2,3-thiadiazole, pyridazine, pyrimidine, purine, and pteridine is preferably used.

The solvent is not particularly limited as long as the solvent is capable of dissolving the metal compound and the organic compound for the organic ligand, and an appropriate solvent can be selected from water and an organic solvent. The solvent is preferably water, an alcohol-based solvent such as methanol, or an amide-based solvent such as dimethylformamide or dimethylacetamide, and at least the amide-based solvent is more preferably included. The structure $S_x$ of the MOF of the present invention is considered to be derived from such a solvent, or water in the air during the production process.

The ratio of the metal compound to the solvent (metal compound/solvent) is preferably 0.01 mol/L or more, more preferably 0.03 mol/L or more, and further preferably 0.05 mol/L or more, and the ratio is preferably 1 mol/L or less, more preferably 0.5 mol/L or less, and further preferably 0.2 mol/L or less.

The ratio of the organic compound for the organic ligand to the solvent (organic compound/solvent) is preferably 0.005 mol/L or more, more preferably 0.01 mol/L or more, and further preferably 0.02 mol/L or more, and the ratio is preferably 3 mol/L or less, more preferably 1 mol/L or less, and further preferably 0.1 mol/L or less.

The molar ratio between the metal compound and the organic compound may be adjusted such that the ratio of the molar quantity of metal atoms in the metal compound to the molar quantity of the organic compound is equal to the above-described molar ratio of the metal ion to the organic ligand in the MOF.

In order to obtain the MOF of the present invention, it is particularly necessary to appropriately set (i) a condition for mixing the metal compound and the organic compound for the organic ligand, and, in addition to the requirement (i), it is also preferable to appropriately adjust at least one of (ii) the temperature at which the mixed compounds are stirred or left as they are, after the metal compound and the organic compound for the organic ligand have been mixed; and (iii) a post-treatment condition for the reaction product of the metal compound and the organic compound for the organic ligand.

Regarding (i), in mixing of the metal compound and the organic compound for the organic ligand, it is important that the metal compound and the organic compound are completely dissolved. Specifically, in the case where the metal compound and the organic compound are mixed in one go, after the mixing, ultrasonic treatment needs to be performed for 2 minutes or more. In addition, in the case where one of the metal compound and the organic compound is completely dissolved in the solvent first, and the other is added thereto, the resulting product, thereafter, needs to be stirred for 5 minutes or more, and, for example, stirring treatment using a stirrer or ultrasonic treatment may be performed.

Regarding (ii), the stirring temperature after mixing the metal compound and the organic compound for the organic ligand may be room temperature or higher, and thereafter, the mixed compounds are preferably left as they are at 100°C to 200°C for approximately 10 hours to 6 days.

Regarding (iii), in the post-treatment condition for the reaction product of the metal compound and the organic compound for the organic ligand, the reaction product is preferably washed three times or more with the solvent used during the reaction of the metal compound and the organic compound, or the like. At this time, the solvent preferably includes, particularly, an amide-based solvent such as dimethylformamide or dimethylacetamide.

The MOF of the present invention can be suitably used to adsorb and remove gas or organic molecules, for example. Examples of the gas include carbon dioxide, hydrogen, carbon monoxide, oxygen, nitrogen, hydrocarbon having 1 to 4 carbon atoms, noble gas, hydrogen sulfide, ammonia, sulfur oxide, nitrogen oxide, and siloxane. Examples of the organic molecule include hydrocarbon having 5 to 8 carbon atoms, alcohol having 1 to 8 carbon atoms, aldehyde having 1 to 8 carbon atoms, carboxylic acid having 1 to 8 carbon atoms, ketone having 1 to 8 carbon atoms, amine having 1 to 8 carbon atoms, ester having 1 to 8 carbon atoms, and amide having 1 to 8 carbon atoms. The organic molecule may contain an aromatic ring.

This application claims the benefit of priority based on the Japanese Patent Application No. 2022-042377 filed on March 17, 2022. The entire contents of the Japanese Patent Application No. 2022-042377 filed on March 17, 2022, are incorporated herein by reference.

EXAMPLES

The present invention will be described in more detail below by means of examples. The present invention. The present invention is not limited by the following examples, and can also be carried out with appropriate modifications being made within the scope of the gist described above and below, and any of these modifications are included in the technical scope of the present invention.

Example 1

In a 50 mL pressure-resistant container (including a Teflon (registered trademark) inner cylinder) made of SUS-304, 3.000 mmol of 2-aminoterephthalic acid was dissolved in 25 mL of a mixture obtained by mixing dimethylformamide (DMF) and MeOH at a ratio of 1/1 (volume ratio), and 1.518 mmol of $Ti[OCH(CH_3)_2]_4$ was mixed with the resulting product at 25°C, and the obtained mixture was stirred for 5 minutes by putting in a stirrer chip. Then, the resulting product was left as it was at 150°C for 16 hours. The obtained precipitated solid product was subjected to three times of washing with 10 mL of DMF and filtering, and the obtained filter cake was dried in a vacuum drying oven at 120°C for 24 hours and at 150°C for 24 hours under reduced pressure, to obtain 0.62 g of a product (yield: 89.4%).

Example 2

In a 50 mL pressure resistant container (including a Teflon (registered trademark) inner cylinder) made of SUS-304, 0.348 mmol of $In(NO_3)_3·5H_2O$, 0.955 mmol of $Mg(NO_3)_2·6H_2O$, 0.518 mmol of 1,3,5-tris(4-carboxyphenyl) benzene, 2.5 ml of $H_2O$, and 13.5 ml of DMF were mixed while being stirred at room temperature, and ultrasonic waves was applied to the mixture for 3 minutes to dissolve. Then, the resulting product was left as it was at 100°C for 5 days. The obtained precipitated solid product was subjected to three times of washing with 10 mL of dimethylacetamide (DMA) and filtering, and the obtained filter cake was dried at room temperature for 3 days, to obtain 0.24 g of a product (yield: 90%).

Example 3

In a 100 mL pressure resistant container (including a Teflon (registered trademark) inner cylinder) made of SUS-304, 0.864 mmol of $Cu(NO_3)_3·3H_2O$, 0.351 mmol of $In(NO_3)_3·5H_2O$, 0.513 mmol of 1,3,5-tris(4-carboxyphenyl)benzene, 2.5 ml of $H_2O$, and 13.5 ml of DMF were mixed while being stirred at room temperature, and ultrasonic waves was applied to the mixture for 2 minutes to dissolve. Then, the resulting product was left as it was at 100°C for 5 days. The obtained precipitated solid product was subjected to three times of washing with 30 mL of DMA and filtering, and the obtained filter cake was dried under reduced pressure in a vacuum drying oven at 120°C for 24 hours, to obtain 0.39 g of a product (yield: 84.9%).

Example 4

In a 100 mL pressure resistant container (including a Teflon (registered trademark) inner cylinder) made of SUS-304, 5.1 mmol of $AlCl_3$, 2.089 mmol of terephthalic acid, and 60 ml of DMF were all mixed, and ultrasonic waves was applied to the mixture at room temperature for 5 minutes to dissolve. Then, the resulting product was left as it was at 110°C for 20 hours. The obtained precipitated solid product was subjected to three times of washing with 30 mL of DMF and filtering, and the obtained filter cake was dried under reduced pressure in a vacuum drying oven at 60°C for 30 minutes and at 150°C for 12 hours, to obtain 0.38 g of a product (yield: 90%).

Comparative Example 1

In a 100 mL pressure resistant container (including a Teflon (registered trademark) inner cylinder) made of SUS-304, 20 ml of tetrahydrofuran (THF) and 1.556 mmol of 2,5-dihydroxyterephthalic acid were mixed, to obtain a mixture A. Separately, 3.028 mmol of $[Zn(NO_3)_2]\cdot 6H_2O$, 6 ml of a NaOH aqueous solution (1 M), and 10 ml of ion-exchanged water were mixed, to obtain a mixture B. The mixture B was added to the mixture A, and the resulting mixture was left as it was at 110°C for 3 days. The obtained precipitated solid product was subjected to three times of washing with 30 ml of THF and filtering, and the obtained filter cake was dried in a vacuum drying oven at 80°C for 24 hours, to obtain 0.16 g of a product (yield: 32.9%).

Comparative Example 2

In a 100 mL pressure resistant container (including a Teflon (registered trademark) inner cylinder) made of SUS-304, 40 ml of N-methylpyrrolidone (NMP) and 3.839 mmol of 2,5-dihydroxyterephthalic acid were mixed, to obtain a mixture A. Separately, 7.605 mmol of $Mn(CH_3COO)_2\cdot 4H_2O$ and 5 ml of ion-exchanged water were mixed, to obtain a mixture B. The mixture B was added to the mixture A, and the resulting mixture was left as it was at 110°C for 24 hours. The obtained precipitated solid product was subjected to three times of washing with 30 ml of ion-exchanged water and filtering, and the obtained filter cake was dried in a vacuum drying oven at 100°C for 24 hours, to obtain 0.66 g of a product (yield: 55.1%).

The products obtained in Examples and Comparative Examples were evaluated by the following methods.

(1) Measurement of decomposition start temperature

Each of the obtained MOFs was held at 25°C for 12 hours under an air atmosphere in which the relative humidity was adjusted to 50% to perform pre-treatment. Then, the temperature was increased to 500°C at a heating rate of 5°C/min. while nitrogen was caused to flow. According to DTA measurement defined in General rules for thermal analysis of JIS K0129, a point at which decomposition behavior was observed in this section (25 to 500°C) was set as a decomposition start temperature.

(2) Evaluation of MOF structure

Under the following condition, XRD (X-ray diffraction) measurement was performed to obtain a crystallographic information file (cif), the cif was loaded into software named Mercury (The Cambridge Crystallographic Data Centre) to obtain an image, and the image was used to calculate the numbers of $O^{2-}$, $OH_2$, and $OH^-$ in one cluster structure, the number of the metal ions in one cluster structure, the number of the metal ions bonded to the oxygen atom of each structure $S_x$, and the ratio between O derived from each structure $S_x$ and O derived from the organic ligand.

Device: SmartLab manufactured by Rigaku Corporation
Ray source: Cu
Measurement range: 2θ=3 to 40°
Step size: 0.01°
Scan speed: 3°/min.
Measurement temperature: room temperature (25°C)

(3) Evaluation of water absorptive performance

Each of the products (MOFs) obtained in Examples and Comparative Examples was left at a temperature of 25°C for 12 hours under an air atmosphere in which the humidity was adjusted so that the relative humidity was 50%, and then a mass $W_1$ was measured. Thereafter, the MOF for which the mass $W_1$ was measured was heated at a heating rate of 5°C/min. while nitrogen was caused to flow, and was left at a temperature of 200°C for 1 hour, and then, a mass $W_2$ was measured. The value obtained by the following formula (1) was set to a water absorption rate A.

$$\text{water absorption rate } A = (W_1 - W_2)/W_2 \qquad (1)$$

(4) Measurement of BET specific surface area

Since the adsorption occupation area of nitrogen molecules is known in advance, the amount of gas molecules adsorbed on only the surface of the sample was measured, and the surface area of the sample was measured according to a BET adsorption isotherm.

Pre-treatment condition: according to the measurement results from TG-DTA described above, the temperature at which water contained in the sample was able to be removed was checked, and heating under reduced pressure was performed overnight.

Device: BELSORP-mini manufactured by MicrotracBEL
Pre-treatment condition:

(1) A glass bar (for a standard sample tube) for reducing the volume was set in a standard sample tube, and the standard sample tube was sealed with a quick seal. The above sample-tube sets, the number of which corresponded to the number of samples to be measured (a maximum of 3 samples per measurement), were prepared and connected to a pre-treatment device (BELPREP VACII). The air inside the sample tube was discharged, and then $N_2$ gas (having a purity of 99.999% or more) was introduced therein until the pressure reached atmospheric pressure;

(2) Thereafter, the sample tube was removed from the pre-treatment device, and the weight was measured by using a precision balance (that displays four or more decimal places) three times to obtain the average value (W1). When the precision balance was used, an ionizer was used to eliminate the effect of static electricity;

(3) About 50 mg of the sample to be measured was weighed on drug packaging paper, and the sample was directly placed into a spherical part in the lower part of the standard sample tube by using a long stem funnel;

(4) The glass bar was returned into the sample tube, the sample tube was sealed with a quick seal, and then the total weight was measured once and the amount of the added sample was tentatively checked;

(5) The sample tube containing the sample was connected to the pre-treatment device to exhaust gas from the sample tube; and

(6) After the pressure in the sample tube became sufficiently low, heating was started (while vacuuming was continued).

Measurement condition:

(1) After the pre-treatment (vacuum heating) was ended, heat was dissipated from the sample tube while the sample tube was held under reduced pressure. After the temperature reached room temperature, $N_2$ gas was introduced therein until the pressure reached atmospheric pressure. The sample tube was removed from the device;

(2) After the pre-treatment, the weight of the sample tube containing the sample was measured three times by using a precision balance to obtain an average value (W2). The weight of the sample was obtained by performing calculation of W2-W1; and

(3) The weight of the sample, and information on $N_2$ gas at a liquid nitrogen temperature (second virial coefficient, and the like) were inputted to the measurement software, the measurement relative pressure at which measurement was desired to be performed was inputted, and a measurement start button was pressed. Then, according to instructions of the software, a Dewar vessel filled with liquid nitrogen or a sample tube was set, and measurement was performed.

The results are shown in Table 1. Each organic ligand in Table 1 is indicated by the name of carboxylic acid in a state before protons are removed from carboxyl groups. In addition, each nuclei number of cluster structure in Table 1 indicates the number of the metal ions in one cluster structure.

[Table 1]

| | Metal ion | Organic ligand | Metal ion/ Organic ligand molar ratio | Decomposition start temperature (°C) | BET specific surface area cm/g$^{2/}$ | Number of structure Sx in one cluster structure* | | | | Cluster structure | $O^2/O$ derived from organic ligand | $OH^-/O$ derived from organic ligand | $OH_2/O$ derived from organic ligand | Water adsorption rate A (wt%) |
|---|---|---|---|---|---|---|---|---|---|---|---|---|---|---|
| | | | | | | $O^{2-}$ | $OH_2$ | $OH^-$ | Total | | | | | |
| Example 1 | Ti | 2-aminotere phthalic acid | 0.5 | 200 | 1388 | 8 (2) | 0 | 4 (2) | 12 | 8 nuclei | 0.33 | 0.17 | 0 | 28.5 |
| Example 2 | In,Mg | 1,3,5-tris(4-car-boxyphenyl)ben-zene | 2.5 | 400 | 1251 | 0 | 4 (2) | 2 (2) | 6 | 4 nuclei (In:2 nu-clei, Mg:2 nuclei) | 0 | 0.13 | 0.25 | 34.7 |
| Example 3 | In,Cu | 1,3,5-tris(4-car-boxyphenyl)ben-zene | 2.4 | 250 | 212 | 0 | 2 (2) | 2 (2) | 4 | 4 nuclei (In:2 nu-clei, Cu:2 nuclei) | 0 | 0.13 | 0.13 | 29.1 |
| Example 4 | Al | Terephthalic acid | 2.4 | 280 | 1259 | 0 | 0 | 2 (2) | 2 | 3 nuclei | 0 | 0.17 | 0 | 27.8 |
| Comparative example 1 | Zn | 2,5-dihydroxytere phthalic acid | 1.9 | 160 | 20 | 0 | 0 | 0 | 0 | N/A | 0 | 0 | 0 | 12.8 |
| Comparative example 2 | Mg | 2,5-dihydroxytere phthalic acid | 2.0 | 160 | 19 | 0 | 0 | 0 | 0 | N/A | 0 | 0 | 0 | 15.8 |
| **\* The number in parentheses indicates the number of metal ions bonded to the oxygen atom of each structure Sx.** | | | | | | | | | | | | | | |

**Claims**

1. A metal organic framework, wherein

   the metal organic framework has a structure $S_{M-x}$,
   in the structure $S_{M-x}$, two or more metal ions are bonded to one oxygen atom in a structure $S_x$ constituted by one or more of $O^{2-}$, $OH_2$, $OH^-$, $OCH_3^-$, and $OC_2H_5^-$, and
   the metal organic framework has a decomposition start temperature of 200°C or higher.

2. The metal organic framework according to claim 1, wherein
   a ratio of O derived from the $OH_2$ to O derived from an organic ligand of the metal organic framework is 0.1 or more.

3. The metal organic framework according to claim 1 or 2, wherein

   the metal organic framework has a cluster structure,
   the cluster structure is constituted by the structure $S_x$ and the metal ions,
   in the cluster structure, the metal ions are bonded to each other via the oxygen atom in the structure $S_x$, and
   the number of the metal ions in one cluster structure is 3 or 4.

4. The metal organic framework according to claim 1 or 2, wherein
   an organic ligand of the metal organic framework includes at least one selected from the group consisting of carboxylates represented by $R(COO^-)_n$ (R represents an n-valent group, and n represents an integer of 2 or more) and an oxalate ion $(COO^-)_2$.

5. The metal organic framework according to claim 1 or 2, wherein
   the metal ion is an ion of at least one metal selected from elements in groups 2 to 14 in the third to sixth periods in a periodic table.

| | INTERNATIONAL SEARCH REPORT | International application No. |
|---|---|---|
| | | **PCT/JP2023/010264** |

**A.    CLASSIFICATION OF SUBJECT MATTER**

*C07C 63/26*(2006.01)i; *C07C 63/331*(2006.01)i; *C07C 229/76*(2006.01)i; *C07F 1/08*(2006.01)i; *C07F 3/02*(2006.01)i; *C07F 5/00*(2006.01)i; *C07F 5/06*(2006.01)i; *C07F 7/28*(2006.01)i
FI:    C07C63/26 Z; C07C229/76; C07C63/331; C07F3/02 Z; C07F5/06 D; C07F5/00 J; C07F1/08 B; C07F7/28 F CSP

According to International Patent Classification (IPC) or to both national classification and IPC

**B.    FIELDS SEARCHED**

Minimum documentation searched (classification system followed by classification symbols)

C07C63/26; C07C63/331; C07C229/76; C07F1/08; C07F3/02; C07F5/00; C07F5/06; C07F7/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus(STN)

**C.    DOCUMENTS CONSIDERED TO BE RELEVANT**

| Category* | Citation of document, with indication, where appropriate, of the relevant passages | Relevant to claim No. |
|---|---|---|
| X | WO 2014/114948 A1 (JOHNSON MATTHEY PUBLIC LIMITED COMPANY) 31 July 2014 (2014-07-31)<br>claims, examples | 1-5 |
| A | JP 2020-132643 A (NTN CORP.) 31 August 2020 (2020-08-31)<br>claims, examples, fig. 2 | 1-5 |
| A | CN 103779597 A (UNIVERSITY OF SCIENCE AND TECHNOLOGY OF CHINA) 07 May 2014 (2014-05-07)<br>claims, examples | 1-5 |
| A | US 2017/0173565 A1 (DOW GLOBAL TECHNOLOGIES LLC) 22 June 2017 (2017-06-22)<br>claims, examples | 1-5 |

☐ Further documents are listed in the continuation of Box C.        ☑ See patent family annex.

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **29 May 2023** | **06 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/010264**

| Patent document cited in search report | | | Publication date (day/month/year) | Patent family member(s) | | | Publication date (day/month/year) |
|---|---|---|---|---|---|---|---|
| WO | 2014/114948 | A1 | 31 July 2014 | GB<br>claims, examples | 2514437 | A | |
| JP | 2020-132643 | A | 31 August 2020 | (Family: none) | | | |
| CN | 103779597 | A | 07 May 2014 | (Family: none) | | | |
| US | 2017/0173565 | A1 | 22 June 2017 | WO<br>claims, examples<br>CN | 2015/175759<br><br>106488803 | A1<br><br>A | |

Form PCT/ISA/210 (patent family annex) (January 2015)

**EP 4 495 098 A1**

REFERENCES CITED IN THE DESCRIPTION

**Patent documents cited in the description**

- JP 2020176101 A

- JP 2022042377 A